(19) Europäisches Patentamt
European Patent Office
Office européen des brevets

(11) Veröffentlichungsnummer : **0 116 341**
**B1**

# (12) EUROPÄISCHE PATENTSCHRIFT

(45) Veröffentlichungstag der Patentschrift : **07.05.86**

(21) Anmeldenummer : **84101041.6**

(22) Anmeldetag : **02.02.84**

(51) Int. Cl.4 : **C 07 D487/04**, G 03 G 5/06// C07D239/50 ,(C07D487/04, 249:00, 239:00)

## (54) Neue 2H-v-Triazolyl(4,5-d)-pyrimidine und deren Verwendung.

(30) Priorität : **09.02.83 DE 3304330**

(43) Veröffentlichungstag der Anmeldung : **22.08.84 Patentblatt 84/34**

(45) Bekanntmachung des Hinweises auf die Patenterteilung : **07.05.86 Patentblatt 86/19**

(84) Benannte Vertragsstaaten : **CH DE FR GB IT LI**

(56) Entgegenhaltungen :
**EP-A- 0 093 330**
**GB-A- 2 040 940**
**US-A- 3 257 204**
**US-A- 4 157 443**

(73) Patentinhaber : **BASF Aktiengesellschaft**
**Carl-Bosch-Strasse 38**
**D-6700 Ludwigshafen (DE)**

(72) Erfinder : **Albert, Bernhard, Dr.**
**Eichenstrasse 60**
**D-6700 Ludwigshafen (DE)**
Erfinder : **Hoffmann, Gerhard, Dr.**
**Pappelstrasse 22**
**D-6701 Otterstadt (DE)**
Erfinder : **Neumann, Peter, Dr.**
**Franz-Schubert-Strasse 1**
**D-6908 Wiesloch (DE)**

**EP 0 116 341 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue 2H-v-Triazolyl [4,5-d]-pyrimidine und deren Verwendung als Ladungsträger transportierende Verbindungen in elektrophotographischen Aufzeichnungsmaterialen.

Elektrophotographische Verfahren, dafür benötigte Materialien und verschiedene Varianten für den Aufbau von Aufzeichnungsmaterialien sind bekannt. Vorteilhaft für den Einsatz im Reproduktionssektor sind Materialien aus polymeren Bindemitteln, die an spezielle Anforderungen des jeweiligen Einsatzgebietes angepaßt werden können, niedermolekularen organischen Verbindungen, die in den Bindemitteln auch in höheren Konzentrationen löslich und zu einem Transport von Ladungsträgers des elektrischen Stromes befähigt sind, sowie Verbindungen, insbesondere Farbstoffe oder Pigmente, die durch Absorption des bildmäßig eingestrahlten, aktinischen Lichtes Ladungsträger des elektrischen Stromes erzeugen und diese unter Mithilfe des von außen durch die elektrostatische Oberflächenladung aufgeprägten elektrischen Feldes auf die Ladung transportierenden Verbindungen übertragen können. Diese Ladungsträger erzeugenden Verbindungen können je nach Einsatzgebiet des Aufzeichnungsmaterials als eigene Schicht innerhalb einer Kompositstruktur eingebracht werden (vgl. DE-OS 22 20 408) oder in Form monodispers gelöster Farbstoffmoleküle in der Mischung aus Bindemittel und Ladungsträger transportierenden Verbindungen vorhanden sein (vgl. DE-PS 1 058 836). Das in der DE-OS 22 20 408 beschriebene mehrlagige elektrophotographische Aufzeichnungsmaterial besteht aus einem elektrisch leitfähigen Trägermaterial, einer ersten, Farbstoff enthaltenden, etwa 0,005 bis 2 μm dicken, durch Belichtung mit aktinischem Licht Ladungsträger des elektrischen Stromes erzeugenden Schicht aus im Dunkeln isolierenden, organischen Materialien mit mindestens einer Ladungen transportierenden Verbindung.

Es ist auch bekannt, photohalbleitende organische Verbindungen zur Herstellung von elektrophotographischen Druckformen und insbesondere elektrophotographischen Offsetdruckformen zu verwenden (vgl. DE-PS 1 117 391 und 1 120 875, DE-AS 15 22 497 und 27 26 116).

Die gestiegenen Anforderungen an Reproduktionssysteme verlangen eine Vielfalt von Aufzeichnungsmaterialien und -systemen, um für spezielle Probleme optimale Lösungen aussuchen zu können. Gewünscht ist eine hohe Lichtempfindlichkeit, eine gute Auflösung und gute Betonerung. Die oft beanstandete ungenügende Betonerung, die auf eine ungünstige Feldstärkedifferenzierung zwischen belichteten und unbelichteten Flächen hinweist, ist hierbei oft auf eine zu hohe Dunkelleitfähigkeit des Aufzeichnungsmaterials im beladenen Zustand zurückzuführen, so daß eine ungenügende Oberflächenladungsdichte vor der aktinischen bildmäßigen Belichtung vorliegt.

Der vorliegenden Erfindung lag die Aufgabe zugrunde, weitere elektrophotographische Aufzeichnungsmaterialien insbesondere für die Herstellung von elektrophotographischen Druckformen wie Offsetdruckformen zu entwickeln, die bei hoher Lichtempfindlichkeit, guter Auflösung und Verarbeitung gleichzeitig ein geringes Dunkelleitvermögen aufweisen.

Es wurde nun gefunden, daß man verbesserte elektrophotographische Aufzeichnungsmaterialien mit elektrisch leitenden Trägern, Ladungsträgern erzeugenden Verbindungen bzw. Sensibilisatoren und Ladungsträger transportierenden Verbindungen erhält, wenn diese Materialien als Ladungsträger transportierende Verbindungen neue 2H-v-Triazolyl [4,5-d]-pyrimidine der Formel (I) enthalten

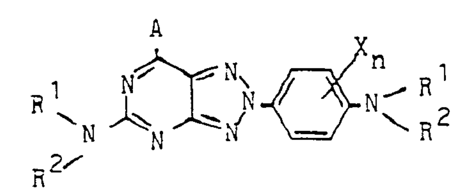

(I)

in der $R^1$ und $R^2$ unabhängig voneinander $C_1$- bis $C_4$-Alkyl, gegebenenfalls durch Chlor, Brom, $C_1$- bis $C_4$-Alkyl oder $C_1$- bis $C_4$-Alkoxy substituiertes Phenyl, Phenalkyl mit insgesamt 7 bis 10 C-Atomen oder

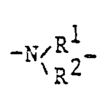

einen gesättigten 5- oder 6-gliedrigen heterocyclischen Ring, und wobei die beiden

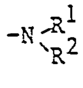

-Gruppen gleich oder verschieden sein können,

X $C_1$- bis $C_4$-Alkyl, Halogen oder Phenyl

n 0, 1 oder 2 und

A 1)

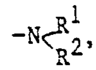

worin $R^1$ und $R^2$ oder

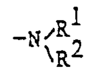

die oben angegebene Bedeutung haben,

2) Wasserstoff, Halogen, Cyan, Thiocyan

3) $C_1$- bis $C_6$-Alkoxy, $C_1$- bis $C_8$-Alkoxy-$C_2$- oder $C_3$-alkoxy, gegebenenfalls durch Cl, Br, $C_1$- bis $C_4$-Alkyl substituiertes Phenoxy, Phenalkoxy mit insgesamt 7 bis 10 C-Atomen, $C_1$- bis $C_{12}$-Alkylthio, gegebenenfalls durch Chlor, Brom, $C_1$- bis $C_4$-Alkyl und/oder $C_1$- bis $C_4$-Alkoxy substituiertes Phenylthio, $—S—(CH_2)_m—COOR^3$

4) $C_1$- bis $C_6$-Alkylcarbonyl, gegebenenfalls durch $C_1$- bis $C_4$-Alkyl oder $C_1$- bis $C_4$-Alkoxy substituiertes Benzoyl, Carboxyl, Carbo-$C_1$- bis $C_8$-alkoxy,

5) $C_1$- bis $C_4$-Alkylsulfonyl, gegebenenfalls durch $C_1$- bis $C_4$- Alkyl substituiertes Phenylsulfonyl

6) $C_5$- bis $C_8$-Cycloalkylamino,

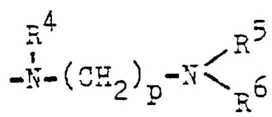 oder 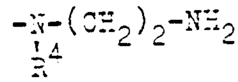

7) 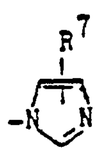 , 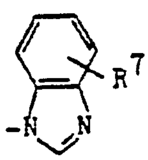 , 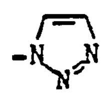 , 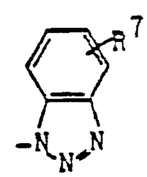

oder

8) 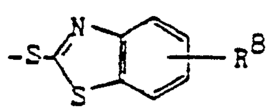 , 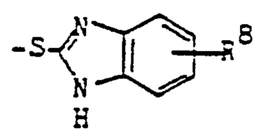 , 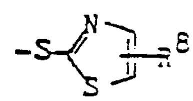

oder 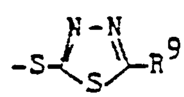

bedeuten und worin

$R^3$ für $C_1$- bis $C_4$-Alkyl,

m für 1, 2, 3 oder 4,

$R^4$ für Wasserstoff oder $C_1$- bis $C_4$-Alkyl,

$R^5$ und $R^6$ unabhängig voneinander für $C_1$- bis $C_4$-Alkyl oder

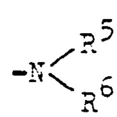

für einen gesättigten 5- oder 6-gliedrigen heterocyclischen Rest, der gegebenenfalls N, O und/oder S als Ringglied enthält,

p für 2, 3, 4 oder 5,

$R^7$ und $R^8$ unabhängig voneinander für Wasserstoff, $C_1$- bis $C_4$-Alkyl, $C_1$- bis $C_4$-Alkylcarbonyl, Nitro, Halogen oder Phenyl

und

$R^9$ für Wasserstoff, $C_1$- bis $C_4$-Alkyl, $C_1$- bis $C_4$-Alkylcarbonyl, Phenyl, Thio oder $C_1$- bis $C_4$-Alkylthio stehen.

Die erfindungsgemäßen elektrophotographischen Aufzeichnungsmaterialien zeichnen sich durch eine Kombination sehr guter Eigenschaften, insbesondere einer hohen Photoleitfähigkeit bei gleichzeitig sehr niedriger Dunkelleitfähigkeit aus, so daß die Schichten für die Kopiertechnik sehr geeignet sind. Deutliche Vorteile weisen sie bei der Verwendung für die Herstellung von elektrophotographischen Druckformen auf und genügen hierbei hohen Ansprüchen im Hinblick auf das Auflösungsvermögen und die Druckauflage.

Als Substituenten $R^1$ und $R^2$ kommen für I z. B. im einzelnen in Betracht :

$C_1$- bis $C_4$-Alkyl : Methyl, Ethyl, n- und i-Propyl, n-Butyl, Isobutyl ; gegebenenfalls substituiertes Phenyl : Phenyl, 2-, 3- und 4-Tolyl, 2-, 3- und 4-Methoxyphenyl, 2-, 3- und 4-Ethoxyphenyl, 2-, 3- und 4-Chlorphenyl, 2-, 3- und 4-Bromphenyl, 3- und 4-Ethylphenyl, 3- und 4-Isopropylphenyl, 3- und 4-sec.-Butylphenyl, Phenalkyl : Benzyl, 2- und 1-Ethylphenyl, 2- und 3-Propylphenyl, Butylphenyl.

Als gesättigte 5- und 6-gliedrige heterocyclische Ringe sind für $—NR^1R^2$ z. B. zu nennen : Reste des Pyrrolidins, Piperidins, Morpholins, Thiomorpholins, Thiomorpholin-S-dioxids und N-$C_1$- bis $C_4$-Alkyl- und N-$C_2$- bis $C_4$-Hydroxyalkylpiperazins, wie N-Methyl-, N-Ethyl-, N-Butyl-N-(β-Hydroxyethyl)-piperazin und N-(β-Hydroxypropyl)-piperazin.

Für $R^1$ und $R^2$ sind Methyl, Ethyl, Phenyl, Benzyl und für

$$-N{<}^{R^1}_{R^2}-$$

Morpholino, Piperidino, Pyrrolidinyl und N'-$C_1$- bis $C_4$-Alkylpiperazino bevorzugt.

Als Substituenten X sind im einzelnen z. B. zu nennen : als $C_1$- bis $C_4$-Alkyl : Methyl, Ethyl, Propyl, Butyl, als Halogen : Brom und Fluor, vorzugsweise Chlor, und Phenyl, wobei n 1 oder 2, vorzugsweise 0 ist.

Für A kommen in Betracht :

1)

$$-N{<}^{R^1}_{R^2},$$

wobei $R^1$ und $R^2$ oder

$$-N{<}^{R^1}_{R^2}$$

die oben angegebenen Bedeutung haben und Cyclohexylamino ;

2) Wasserstoff ; Halogen wie Brom, Fluor, vorzugsweise Chlor, Cyan oder Thiocyanat ;

3) a) $C_1$- bis $C_6$-Alkoxy : Methoxy, Ethoxy, n- und i-Propoxy, n-Butoxy, n-Pentoxy, Hexoxy, 2,2-Dimethylpropoxy ;

b) $C_1$- bis $C_8$-Alkoxy-$C_2$- oder $C_3$-alkoxy : 2-Methoxyethoxy, 2-Ethoxyethoxy, 2-Propoxyethoxy, 2-Butoxyethoxy, 2-Hexoxyethoxy, 2-Octoxyethoxy, 2-(2'-Ethylhexoxy)-ethoxy, 3-Methoxypropoxy, 3-Ethoxypropoxy, 3-Propoxypropoxy, 3-Butoxypropoxy, 3-Hexoxypropoxy, 3-(2'-Ethylhexoxy)-propoxy, 3-Octoxypropoxy ;

c) gegebenenfalls substituiertes Phenoxy : Phenoxy, 3- und 4-Chlorphenoxy, 3- und 4-Bromphenoxy, 3- und 4-Methylphenoxy, 3- und 4-Ethylphenoxy,

d) Phenalkoxy : Benzyloxy, 2-Phenylethoxy, 2- und 3-Phenylpropoxy ;

e) $C_1$- bis $C_{12}$-Alkylthio : Methyl-, Ethyl-, Propyl-, Butyl-, Hexyl-, Octyl-, Decyl- und Dodecylmercapto

f) gegebenenfalls substituiertes Phenylthio : Phenylthio, 3- und 4-Chlorphenylthio, 4-Methylthio, 3- und 4-Bromphenylthio, 3- und 4-Methoxyphenylthio, 3- und 4-Ethoxyphenylthio,

g) $—S—(CH_2)_m—COOR^3$ : m = 1, 2, 3 oder 4 und $R^3$ $C_1$- bis $C_4$-Alkyl wie Methyl, Ethyl, Propyl und Butyl.

4) Neben Carboxyl als Alkylcarbonyl und gegebenenfalls substituiertes Benzoyl : Acetyl, Propionyl, Butyryl, Benzoyl, 4-Methylbenzoyl, 4-Methoxybenzoyl und als Carboalkoxy : Carbomethoxy, Carboethoxy, Carbopropoxy und Carbobutoxy.

5) Alkylsulfonyl : Methylsulfonyl, Ethylsulfonyl, Phenylsulfonyl, p-Tolylsulfonyl.

6) Reste der Formel

$$-\overset{R^4}{\overset{|}{N}}-(CH_2)_p-N{<}^{R^5}_{R^6},$$

in der $R^4$ für Wasserstoff oder $C_1$- bis $C_4$-Alkyl und $R^5$, $R^6$ je für $C_1$- bis $C_4$-Alkyl oder

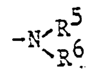

für die für

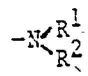

angegebenen Gruppen stehen,
sowie

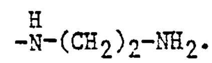

7) und 8)

Reste der unter 7) und 8) auf Seite 3 angegebenen Formeln, in denen $R^7$, $R^8$ und $R^9$ die oben angegeben Bedeutung haben.

Von den für A unter 1) bis 7) genannten Substituenten sind aus anwendungstechnischen Gründen bevorzugt:

1.1) für $R^1$ und $R^2$ in

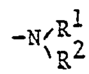

Methyl, insbesondere Ethyl und für

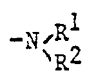

Morpholinyl, Piperidinyl, Pyrrolidinyl, N'-$C_1$- bis $C_4$-Alkylpiperazinyl;

2.1) Wasserstoff und Chlor,

3.1) $C_1$- bis $C_6$-Alkoxy, insbesondere Methoxy, Ethoxy, n-Propoxy, n-Butoxy und 2,2-Dimethylpropoxy; 2-$C_1$- bis $C_4$-Alkoxyethoxy, insbesondere 2-Methoxyethoxy; Phenoxy; Benzyloxy; $C_1$- bis $C_{12}$-Alkylthio, insbesondere Dodecylthio, Butylthio; Thiophenyl und $—S—(CH_2)_m—COOR^3$ mit $m = 2$ und $R^3 = C_1$- bis $C_4$-Alkyl, insbesondere Methyl und Ethyl;

4.1) Acetyl, Propionyl, Benzoyl, Carbomethoxy und Carboethoxy;

5.1) Benzolsulfonyl;

6.1) $—NH—(CH_2)_2—NH_2$ und

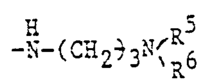

mit $R^5$ und $R^6$ = Methyl und Ethyl;

7.1) Imidazolyl-(1), Benzimidazolyl-(1) und Benzthiazolyl-2-thio, wobei die Phenylreste gegebenenfalls durch Nitro substituiert sind.

Besonders bevorzugt sind wegen der hervorragenden anwendungstechnischen Eigenschaften Triazolylpyrimidine der Formel II

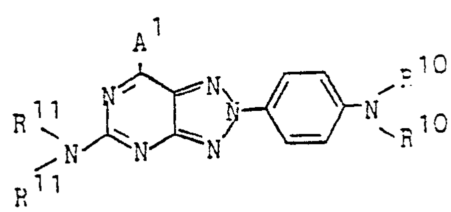

(II)

in der $A^1$ Brom, Chlor, $—S—(CH_2)_2—COOC_1$- bis $C_4$-Alkyl, N-Morpholino, N-Piperidino, N-Cyclohexylamino, N'-$C_1$- bis $C_4$-Alkylpiperazino, Imidazolyl-(1) oder $C_1$- bis $C_6$-Alkoxy und $R^{11}$ und $R^{12}$ unabhängig voneinander $C_1$- bis $C_4$-Alkyl, insbesondere Methyl oder Ethyl oder Phenyl bedeuten, und worin $R^{11}$ und $R^{12}$ gleich oder verschieden sein können.

Ganz besonders sind solche Verbindungen (II) bevorzugt, in denen $A^1$ für Chlor-, $—S—(CH_2)—COOCH_3$, N-Morpholino, N-Piperidino, N-Cyclohexylamino, N'-Methylpiperazino, Imidazolyl-(1), Methoxy, Ethoxy, n-Propoxy, n-Butoxy oder 2,2-Dimethylpropoxy und

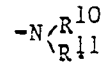

unabhängig voneinander für Dimethylamino, Diethylamino oder Diphenylamino stehen.

Von den Verbindungen der Formel II sind solche der Formel III ganz besonders hervorzuheben :

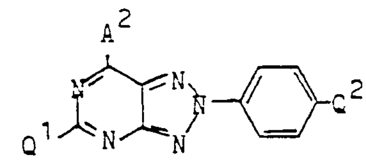

(III)

| | $A^2$ | $Q^1$ | $Q^2$ |
|---|---|---|---|
| (III a) | $-Cl$ | $-N(C_2H_5)_2$ | 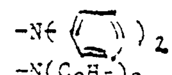 |
| (III b) | $-Cl$ | $-N(CH_3)_2$ | $-N(C_2H_5)_2$ |
| (III c) | $-Cl$ | $-N(C_2H_5)_2$ | $-N(C_2H_5)_2$ |
| (III d) | $-S-C_2H_4-\overset{O}{\overset{\|}{C}}OCH_3$ | $-N(C_2H_5)_2$ | $-N(C_2H_5)_2$ |
| (III e) | $-OCH_3$ | $-N(C_2H_5)_2$ | $-N(C_2H_5)_2$ |
| (III f) | $-OC_2H_5$ | $-N(C_2H_5)_2$ | $-N(C_2H_5)_2$ |
| (III g) | $-OCH_2-CH_2-CH_3$ | $-N(C_2H_5)_2$ | $-N(C_2H_5)_2$ |
| (III h) | $-O-(CH_2)_3CH_3$ | $-N(C_2H_5)_2$ | $-N(C_2H_5)_2$ |
| (III i) | $-O-CH_2-C(CH_3)_3$ | $-N(C_2H_5)_2$ | $-N(C_2H_5)_2$ |
| (III k) | $-O-CH_2-CH_2-O-CH_3$ | $-N(C_2H_5)_2$ | $-N(C_2H_5)_2$ |
| (III l) | $-N(C_2H_5)_5$ | $-N(C_2H_5)_2$ | $-N(C_2H_5)_2$ |

Von diesen Verbindungen sind die der Formeln (III 1) und vor allem die der Formel (III c) und (III e) hervorzuheben.

Die neuen Triazolyl [-4,5-d]-pyrimidine I können nach an sich bekannten Verfahren hergestellt werden.

So kann (I) auf folgendem Wege hergestellt werden :

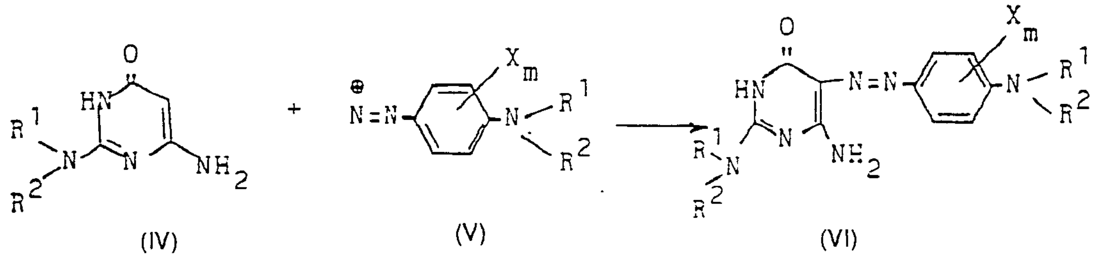

(IV) (V) (VI)

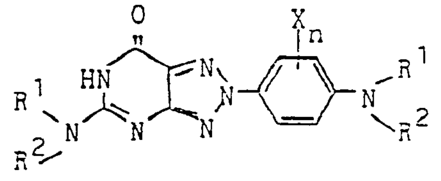

,

(VII)

wobei in den Formeln R¹, R², X und n die oben angegebene Bedeutungen haben. Bei dem Verfahren wird der durch Kupplung von (V) auf (IV) erhaltene o-Aminoazofarbstoff oxidativ in das Triazolderivat überführt.

Die Kupplung von (VI) auf (VI) erfolgt in der Regel bei Temperaturen von — 10 bis + 20 °C, vorzugsweise bei 0 bis 10 °C.

Für die Oxidation von (VI) zu (VII) kommen als Oxidationsmittel die verschiedensten in Betracht, z. B. Chromsäure, Alkalidichromate, Wasserstoffperoxid, Bleitetraacetat, Kaliumferricyanid, Ferrichlorid und Kupfer-(II)-sulfat. In sauren Lösungsmitteln, z. B. wäßriger Essigsäure, werden vorzugsweise Alkalidichromate, Wasserstoffperoxid oder Bleitetraacetat und in basischen Lösungsmittel, z. B. Pyridin-Wassergemisch, vorzugsweise Kaliumferricyanid, verwendet. Der oxidative Ringschluß wird vorzugsweise mit Kupfer-(II)-sulfat in einem Pyridin-Wassergemisch bewirkt. Die Oxidation mit Kupfer-(II)-salzen, wie Kupfer-(II)-sulfat oder -chlorid lät sich mit Vorteil auch in Methanol oder Methanol-Wassergemischen in Gegenwart von Ammonium- oder Aminsalzen, wie Mono- oder Dialkanolaminen durchführen. Der oxidative Ringschluß wird bei einer Temperatur von 70 bis 100 °C, vorzugsweise 90 bis 100 °C durchgeführt.

In der erhaltenen Oxyverbindung (VII) wird dann nach üblichen Verfahren der Sauerstoff durch Chlor ersetzt :

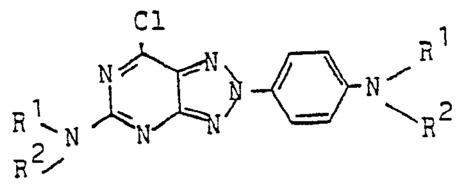

(VIII)

Hierzu können unterschiedliche Chlorierungsmittel angewendet werden, z. B. Phosphoroxychlorid, gegebenenfalls in Gegenwart von tertiären organischen Basen wie Triethylamin, Diethylanilin, Phosphorpentachlorid oder Thionylchlorid, z. B. in Gegenwart von Dimethylformamid.

Die Chlorverbindung (VIII) kann dann nach an sich bekannten Verfahren durch Umsetzen mit Verbindungen der Formel AM (IX), in der M Wasserstoff oder ein Äquivalent eines Metalls ist und A die oben angegebene Bedeutung hat, in geeigneten Lösungsmitteln, gegebenenfalls in Gegenwart von Säureacceptoren, in die Verbindungen der Formel (I), in der A von Chlor verschieden ist, überführt werden.

Als Lösungsmittel kommen hierfür z. B. Methylenchlorid, Chlorbenzol, Tetrahydrofuran, Dimethylformamid, N-Methylpyrrolidon oder Gemische davon in Betracht.

Bei dem Verfahren kommen als Säureacceptoren vorzugsweise tertiäre Amine, wie Triethylamin, Tripropylamin, Tributylamin, in Betracht.

Die Herstellung von Triazolylpyrimidinen der Formel (I) wird durch die Ausführungsbeispiele erläutert.

Die neuen Triazolpyrimidine der Formeln (I), (II) und (III) sind hervorragend als Ladungsträger transportierende Verbindungen in elektrophotographischen Schichten geeignet. Die neuen Verbindungen können mit Vorteil sowohl in einschichtigen als auch in mehrschichtigen auf elektroleitfähige Träger aufgebrachten Aufzeichnungssystemen verwendet werden.

Für diesen Verwendungszweck haben sich die Verbindungen der Formel (II) und insbesondere die Verbindungen der Formel (III) bewährt. Von den letzteren sind die Verbindungen der Formeln (IIIa) bis (IIIc) und (IIIe) ganz besonders hervorzuheben.

Geeignete einschichtige Systeme weisen bevorzugt auf einem leitfähigen Trägermaterial eine Schicht aus (a) 45 bis 75 Gewichtsteilen eines Bindemittels, (b) 30 bis 60, insbesondere 35 bis 50 Gewichtsteilen einer der erfindungsgemäß verwendeten, Ladungsträger transportierenden Verbindungen, (c) gegebenenfalls 5 bis 25 Gewichtsteilen eines weiteren, im wesentlichen inaktiven Bindemittels und (d) 0,05 bis 0,8 Gewichtsteilen einer bei aktinischer Belichtung Ladungsträger erzeugenden Verbindung, insbesondere eines geeigneten Farbstoffs auf. Die Schichten werden mit Vorteil aus einer ca. 5 gew.%igen Lösung in einem geeigneten organischen Lösungsmittel auf das gereinigte leitfähige Trägermaterial so aufgebracht, daß nach dem Ablüften des Lösungsmittels eine Trockenschichtdicke von ca. 0,8 bis 40 µm (je nach Verwendungszweck, bei elektrophotographischen Druckformen insbesondere 0,8 bis 6 µm) resultiert.

Geeignete Mehrschichtsysteme haben auf einem elektroleitfähigen Trägermaterial z. B. (a) eine Ladungsträger erzeugende Schicht und (b) eine Ladungstransportschicht aus 30 bis 60 Gewichtsteilen mindestens einer Ladungsträger transportierenden Verbindung der Formel (I), 45 bis 75 Gewichtsteilen eines organischen Bindemittels und gegebenenfalls 5 bis 25 Gewichtsteilen weiterer, die mechanischen Eigenschaften der Schicht verbessernde Zusätze. Die erste Schicht wird vorteilhaft in einer Dicke von 0,005 bis 5 µm, insbesondere 0,1 bis 0,9 µm als Lösung in einem geeigneten Lösungsmittel auf das Trägermaterial aufgetragen. Nach dem Auftrag erfolgt der Auftrag der zweiten Schicht in einer Dicke, daß nach dem Trocknen der Kompositstruktur eine Schichtdicke von 5 bis 25, insbesondere 7 bis 15 µm resultiert.

Als elektrisch leitende Träger sind prinzipiell alle leitfähigen Trägermaterialien verwendbar, soweit sie für das Einsatzgebiet geeignet sind. Bevorzugt sind je nach Einsatzgebiet der Aufzeichnungsmaterialien Aluminium-, Zink, Magnesium-, Kupfer- oder Mehrmetallplatten, z. B. rohe oder vorbehandelte, z. B. aufgerauhte und/oder anodisierte Aluminiumbleche, Aluminiumfolien, Polymerfilme mit metallisierter Oberfläche wie aluminiumbedampfte Polyethlenterephthalatfilme oder auch elektrisch leitende Spezialpapiere. Träger für Druckformen haben vorteilhaft eine Dicke von 0,08 bis ca. 0,3 mm.

Die Art der geeigneten organischen Bindemittel für die Schichten richtet sich nach dem beabsichtigten Verwendungsweck der Aufzeichnungsmaterialien. Für den Kopiersektor eignen sich z. B. Celluloseether, Polyesterharze, Polyvinylchloride, Polycarbonate, Copolymere, wie Styrol-Maleinsäureanhydrid-Copolymere oder Vinylchlorid-Maleinsäureanhydrid-Copolymere oder Mischungen solcher Bindemittel. Bei ihrer Auswahl spielen ihre filmbildenden und elektrischen Eigenschaften, ihre Haftfestigkeit auf dem Trägermaterial und ihe Löslichkeitseigenschaften eine besondere Rolle. Insbesondere bei Aufzeichnungsmaterialien für die Herstellung elektrophotographischer Druckplatten und besonders bei denen für den Offsetdruck sind solche besonders geeignet, die in basischen wäßrigen oder alkoholischen Lösungsmitteln löslich sind. Dies sind vor allem Substanzen mit alkalilöslich machenden Gruppen wie Anhydrid-, Carboxyl-, Sulfonsäure-, Phenol- oder Sulfonimid-Gruppierungen. Bevorzugt sind Bindemittel, insbesondere solche mit hohen Säurenzahlen, die in basischen wäßrig-alkoholischen Lösungsmittelsystemen leicht löslich sind und ein mittleres Molekulargewicht (Gewichtsmittel) von 800 bis 80 000 und insbesondere 1 500 bis 50 000 aufweisen. Geeignet sind z. B. Copolymerisate aus Methacrylsäure und Methacrylsäureestern, besonders Copolymerisate aus Styrol und Maleinsäureanhydrid und aus Styrol, Methacrylsäure und Methacrylsäureester, soweit sie die vorstehende Löslichkeitsbedingung aufweisen. Obwohl bekanntermaßen Bindemittel mit freien Carboxylgruppen die Dunkelleitfähigkeit der elektrophotographischen Schichten in unerwünschter Weise erhöhen und dadurch zu schlechten Betonerungsergebnissen führen, lassen sich solche Bindemittel leicht an die erfindungsgemäß verwendeten Benztriazole anpassen. So hat sich gezeigt, daß Copolymerisate aus Styrol, Maleinsäureanhydrid und Acryl- oder Methacrylsäure, die einen Anteil von einpolymerisiertem Maleinsäureanhydrid von 5 bis 50 Gew.% und einen Anteil von einpolymerisierter Acryl- oder Methacrylsäure von 5 bis zu 35 und insbesondere 10 bis 30 Gew.% aufweisen, befriedigende elektrophotographische Schichten mit hinreichender Dunkelleitfähigkeit ergeben. Sie weisen eine hervorragende Löslichkeit in Auswaschmitteln aus 75 Gew.% Wasser, 23 Gew.% Isobutanol und 2 Gew.% Soda auf, sind aber in offsettypischem Wischwasser unlöslich.

Geeignete Ladungsträger erzeugende Verbindungen bzw. Sensibilisatoren sind z. B. für einschichtig aufgetragene Systeme, wie sie auch zur Herstellung elektrophotographischer Druckformen dienen, Farbstoffe aus der Triarlymethanreihe, Xanthenfarbstoffe und Cyaninfarbstoffe. Sehr gute Ergebnisse wurden mit den erfindungsgemäßen Verbindungen der Formel I und Rhodamin B (C.I. 45170), Rhodamin 6 G (C.I. 45160), Malachitgrün (C.I. Basic Green 4 ; C.I. 4200) Methylviolett (C.I. 42535) oder Kristalliegt der Farbstoff oder das Pigment in einer separaten Ladungsträger erzeugenden Schicht vor. Hier sind Azofarbstoffe, Phthalocyanine, Isoindolinfarbstoffe und Perylentetracarbonsäurederivate besonders wirksam. Gute Ergebnisse werden mit Perylen-3,4 : 9,10-tetracarbonsäurediimidderivaten erzielt, wie sie in den DE-OS 31 10 954 und 31 10 960 beschrieben sind.

Für die jeweilige Verwendung kann das erfindungsgemäße elektrophotographische Aufzeichnungsmaterial übliche Zusätze enthalten, z. B. Verlaufmittel und Weichmacher in der photoleitfähigen Schicht oder Haftvermittler zwischen Träger und Schicht.

Die erfindungsgemäßen elektrophotographischen Aufzeichnungsmaterialien zeichnen sich durch eine Kombination sehr guter Eigenschaften, insbesondere einer hohen Photoleitfähigkeit bei gleichzeitig sehr niedriger Dunkelleitfähigkeit aus, so daß die Schichten für die Kopiertechnik sehr geeignet sind.

Deutliche Vorteile weisen diese Materialien bei der Verwendung für die Herstellung von elektrophotographischen Druckformen auf und genügen hierbei hohen Ansprüchen im Hinblick auf das Auflösungsvermögen und die Druckauflage. Die hohe Lichtempfindlichkeit erlaubt eine Senkung der Belichtungszeit bei der Verarbeitung in der Reprokamera gegenüber handelsüblichen Materialien bis etwa um die Hälfte. Aus einer sehr randscharfen Bildwiedergabe resultiert eine gute Auflösung. Durch einen hohen Ladungskontrast können auch feine Rasterpunkte in den lichten Tonwortbereichen gut wiedergegeben werden. Ferner führt die Belichtung der Schichten zu sehr geringen Restspannungen und die bei der Betonerung erhaltenen Bilder zeichnen sich durch gute Grundfreiheit in den Nichtbildbereichen aus. Die spektrale Empfindlichkeit sinkt bei 600 nm stark ab, so daß die Schichten bei Rotlicht gehandhabt werden können, ohne daß Bildverluste auftreten.

Die Herstellung elektrophotographischer Offsetdruckformen erfolgt dabei wie üblich durch eine elektrostatische Aufladung des elektrophotographischen Aufzeichnungsmaterials mittels einer Hochspannungscorona, eine direkt nachfolgende bildmäßige Belichtung, die Entwicklung des vorliegenden elektrostatischen, latenten Ladungsbildes mittels eines Trocken- oder Flüssigtoners, die Fixierung des Toners durch einen nachgeschalteten Schmelzvorgang und die Entfernung des unbetonerten, photohalbleitenden Schicht mittels eines geeigneten Auswaschlösemitttels. Die so erhaltene Druckform kann in bekannter Weise für den Offsetdruck noch vorbereitet werden, z. B. durch eine Hydrohilierung und Gummierung der wasserführenden Oberfläche.

Die folgenden Anwendungsbeispiele sollen die erfindungsgemäße Anwendung von Triazolylpyrimidinen der Formel (I) zusätzlich erläutern und belegen.

Die genannten Teile (T.) und Prozentangaben beziehen sich auf das Gewicht.

## I. Herstellung der Triazolylpyrimidine

### Beispiel 1

1.1 Zu einer Lösung von 200,5 T. N,N-Diethyl-p-phenylendiamin-hydrochlorid in 250 T. konzentrierter

Salzsäure und 500 T. Wasser gibt man unter Rühren bei 0 bis 5 °C eine Lösung von 69 T. Natriumnitrit in 160 T. Wasser. Dauer : 30 min. Nach 30 min gibt man etwas Harnstoff zu und tropft die erhaltene Lösung des Diazoniumsalzes bei 5 bis 10 °C unter Rühren zu einer Suspension von 182 T. 2-Diethylamino-4-aminopyrimidon-(6) in 1 450 T. konzentrierter Salzsäure und 1 250 T. Wasser. 30 min. nach beendeter Zugabe tropft man 50 %ige Natronlauge zu, bis der pH-Wert 6 erreicht, rührt über Nacht und saugt die Fällung ab. Das Filtergut wird mit Wasser gewaschen und im Vakuum getrocknet.

Ausbeute : 286 T. Verbindung der Formel (VI) mit $R^1 = R^2 = —C_2H_5$ und m = 0.

1.2 Zu 36 T. der nach 1.1 erhaltenen Azoverbindung in 160 T. Pyridin tropft man bei 70 °C eine Lösung von 75 T. $CuSO_4 \cdot 5H_2O$ in 320 T. Wasser. Die Temperatur wird dann langsam auf Rückflußtemperatur erhöht, 5 h bei dieser Temperatur gehalten und während dieser Zeit Luft durch die Lösung geleitet. Nach dem Abkühlen wird die Fällung abgesaugt, mit Wasser gewaschen und getrocknet (Vakuum).

Ausbeute : 25 T. Verbindung (VII) mit $R^1 = R^2 = —C_2H_5$ und m = 0. Schmelzpunkt 242 °C (umkrist. Toluol).

Analyse : $C_{18}H_{25}N_7O$ (M. 355)

| | | | | |
|---|---|---|---|---|
| ber. | C 60,83 | H 7,09 | N 27,58 | O 4,50 % |
| gef. | C 60,5 | H 7,1 | N 28,5 | O 4,7 %. |

1.3 Zu einer Suspension von 30,2 T. des nach 1.2 erhaltenen Pyrimidons in 250 T. Chlorbenzol tropft man bei 10 °C 78,3 T. Phosphoroxychlorid. Nach der Zugabe erhöht man die Temperatur langsam auf 50 °C und hält 6 h bei dieser Temperatur. Dann destilliert man den Überschuß unter Vakuum (Wasserstrahlpumpe) ab, wobei das abdestillierende Oxychlorid durch die gleiche Menge Chlorbenzol ersetzt wird, so daß das Flüssigkeitsvolumen im Kolben nicht verringert wird. Nachdem das Oxychlorid entfernt ist, wird das Gemisch mit 200 T. Methylenchlorid verdünnt und die Lösung mit eiskaltem Wasser gewaschen. Nach dem Trocknen über Magnesiumsulfat entfernt man die Lösungsmittel zuletzt in Vakuum. Es verbleibt ein dunkelgelbes Öl, das beim Stehen langsam kristallin wird.

Ausbeute : 25 T. Verbindung (II) mit $A^1$ = Cl und
$R^{10} = R^{11} = —C_2H_5$ (III c)
Schmelzpunkt : 124 bis 126 °C.

Beispiel 2

Zu einer Lösung von 10,6 T. der nach 1.3 erhaltenen Chlorverbindung in 135 T. Methylenchlorid und 3,6 T. Triethylamin tropft man bei Raumtemperatur 6,3 T. N-Methylpiperazin in 27 T. Methylenchlorid und rührt 5 h bei 40 °C. Nach dem Abkühlen schüttelt man zweimal mit Wasser aus, trocknet und destilliert das Lösungsmittel ab. Dabei bleibt ein dunkelgelbes Öl zurück, das langsam kristallisiert.

Ausbeute : 9,3 T. der Verbindung der Formel (II) mit

$A^1$ = 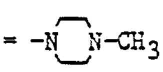

und $R^{10} = R^{11} = C_2H_5$
Schmelzpunkt : 94 bis 95 °C
Analyse : $C_{23}H_{35}N_9$ (M 437)

| | | | |
|---|---|---|---|
| ber. | C 63,13 | H 8,06 | N 28,81 % |
| gef. | C 63,2 | H 8,2 | N 28,1 %. |

Beispiel 3

Zu 10,6 T. der nach 1.3 erhaltenen Chlorverbindung in 120 T. Methanol tropft man 10,2 T. einer 30 %igen methanolischen Natriummethylatlösung und rührt 5 h bei 50 °C. Das Reaktionsgemisch engt man in Vakuum ein, gibt 270 T. Methylenchlorid zu und schüttelt das Gemisch mehrmals mit Wasser aus. Nach dem Trocknen ($MgSO_4$) entfernt man das Lösungsmittel im Vakuum.

Ausbeute : 11,5 T. Methoxyverbindung der Formel (II) mit $A^1 = OCH_3$ und $R^{10} = R^{11} = —C_2H_5$ (IIIe).
Schmelzpunkt : 103 bis 105 °C
Analyse : $C_{19}H_{27}N_7O$ (M 369)

| | | | | |
|---|---|---|---|---|
| ber. | C 61,77 | H 7,37 | N 26,54 | O 4,33 % |
| gef. | C 61,9 | H 7,5 | N 26,2 | O 4,2 %. |

Beispiel 4

Zu einer Lösung von 8,9 T. der nach 1.3 erhaltenen Chlorverbindung in 200 T. Methylenchlorid gibt man 3,5 T. Triethylamin und tropft in 15 min. eine Lösung von 7,8 T. β-Mercaptopropionsäuremethylester

in 30 T. Methylenchlorid zu. Nach dem Rühren bei Raumtemperatur über Nacht wird das Reaktionsgemisch zweimal mit Wasser ausgeschüttet und die organische Phase nach dem Trocknen im Vakuum eingeengt. Das erhaltene Öl wird zu Reinigung mit Toluol/Essigsäureethylester (4 : 1) an Kieselgel chromatographiert.

Ausbeute : 3,5 T. der Verbindung der Formel (II) mit

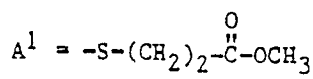

und $R^{10} = R^{11} = —C_2H_5$ (IIId).

Schmelzpunkt : 72 bis 74 °C

Beispiel 5

Zu einer Lösung von 10,6 T. der nach Beispiel 1.3 erhaltenen Chlorverbindung in 200 T. Methylenchlorid und 50 T. Dimethylformamid werden 3,5 T. Triethylamin und 9,3 T. 5(6)-Nitrobenzimidazol gegeben und das Gemisch 8 h auf Rückflußtemperatur erwärmt. Nach dem Abkühlen schüttelt man mit Wasser aus, trocknet die organische Phase mit $MgSO_4$ und engt ein.

Das Rohprodukt wird aus Isobutanol umkristallisiert.

Ausbeute : 5,4 T. Verbindung der Formel (II) mit

$A^1$ =

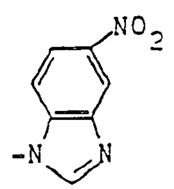

und

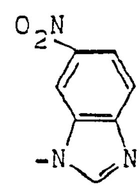

und $R^{10} = R^{11} = —C_2H_5$.

Schmelzpunkt : 218 bis 220 °C

Beispiel 6

6.1 Man verfährt wie in Beispiel 1.1 angegeben, verwendet jedoch bei der Diazotierung 51 T. 4-Aminotriphenylamin und als Kupplungskomponente 30 T. 2-Dimethylamino-4-amino-pyrimidon-(5).

Man erhält die Azoverbindung der Formel

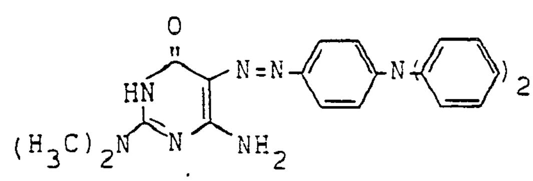

6.2 Analog Beispiel 1.2 erhält man aus der nach 6.1 hergestellten Azoverbindung das entsprechende Hydroxytriazolylpyrimidin der Formel

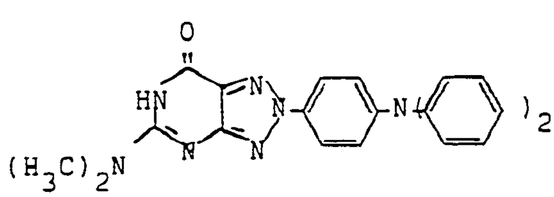

Schmelzpunkt : 282 bis 285 °C.

6.3 Analog der in Beispiel 1.3 beschriebenen Arbeitsweise erhält man mit der Oxyverbindung aus 6.2 2-(4'-Diphenylamino-phenyl)-5-dimethylamino-7-chlor-triazolo-[4,5-d]-pyrimidin.

Schmelzpunkt : 165-167 °C.

Beispiel 7

7.1 Man verfährt wie im Beispiel 1.1 angegeben, verwendet jedoch 43 T. 4-Aminotriphenylamin als Diazokomponente. Man erhält die Azoverbindung der Formel

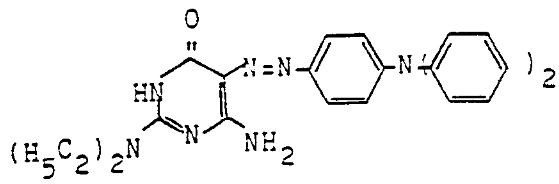

7.2 Entsprechend den Angaben in 1.2 erhält man mit der nach 7.1 erhaltenen Azoverbindung das Oxytriazolylpyrimidin der Formel

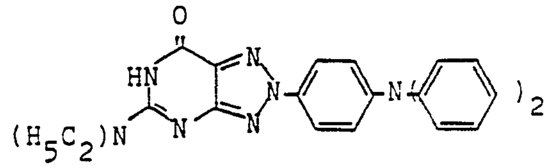

Schmelzpunkt : 262 bis 264 °C.

7.3 Das nach 7.2 erhaltene Oxytriazolylpyrimidin wird nach den Angaben des Beispiels 1.3 in die Chlorverbindung der Formel

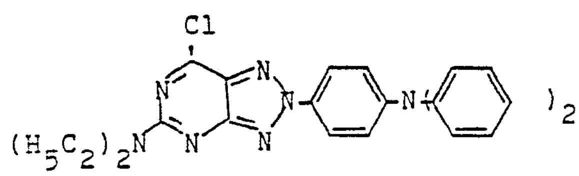

(IIIa)

überführt.

Schmelzpunkt : 139 bis 141 °C.

Beispiel 8

8.1 Man arbeitet wie im Beispiel 1.1, verwendet jedoch als Kupplungskomponente 154 T. 2-Dimethylamino-4-oxy-6-aminopyrimidin.

Ausbeute : 270 T. Azoverbindung der Formel

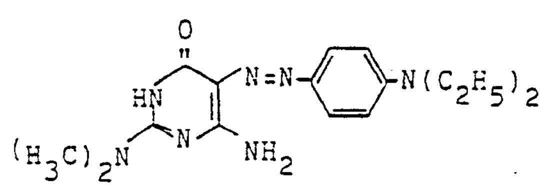

8.2 Nach dem Beispiel 1.2 beschriebenen Verfahren erhält man aus der nach 8.1 hergestellten Azoverbindung das Triazolylpyrimidin der Formel

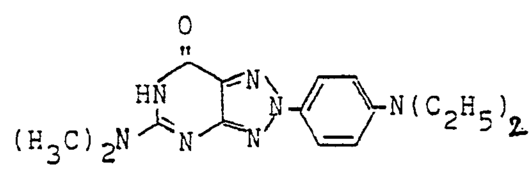

8.3 Das nach 8.2 erhaltene Oxytriazolylpyrimidin wird entsprechend den Angaben in Beispiel 1.3 in die 7-Chlorverbindung überführt.

Schmelzpunkt : 165 bis 167 °C.

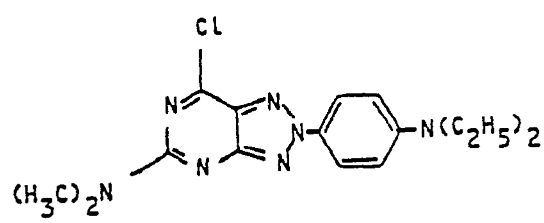

(IIIb)

Beispiele 9 bis 46

Entsprechend den Beispielen 2 bis 5 wurden die nach den Beispielen 1, 6, 7 oder 8 hergestellten 7-Chlor-triazolylpyrimidine zu Verbindungen der Formel

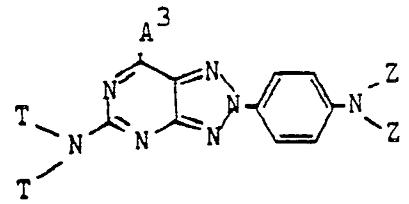

(X)

umgesetzt. Die Bedeutung von $A^3$, Z und T ist in der folgenden Tabelle angegeben :

| Beispiel | T | Z | $A^3$ | Schmelzpunkt [°C] |
|---|---|---|---|---|
| 9 | $-C_2H_5$ | $-C_2H_5$ | $-S-(CH_2)_{11}-CH_3$ | 49- 51 |
| 10 | $-C_2H_5$ | $-C_2H_5$ | 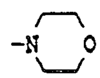 | 158-159 |
| 11 | $-C_2H_5$ | $-C_2H_5$ | 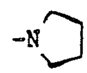 | 170-171 |
| 12 | $-C_2H_5$ | $-C_2H_5$ | $-N(C_2H_5)_2$ | 105-107 |
| 13 | $-C_2H_5$ | $-C_2H_5$ | $-N(-CH_2-\underset{C_4H_9(n)}{CH}-C_2H_5)_2$ | Öl |
| 14 | $-C_2H_5$ | $-C_2H_5$ | $-\underset{H}{N}-CH_2-CH=CH_2$ | 136-138 |
| 15 | $-C_2H_5$ | $-C_2H_5$ | $-S-(CH_2)_2COOCH_3$ | 72- 74 |
| 16 | $-C_2H_5$ | $-C_2H_5$ | 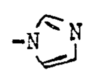 | 163-164 |
| 17 | $-CH_3$ | $-C_2H_5$ | 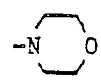 | 191-192 |
| 18 | $-CH_3$ | $-C_2H_5$ | 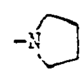 | 169-170 |
| 19 | $-CH_3$ | $-C_2H_5$ | 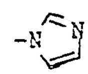 | 163-164 |
| 20 | $-CH_3$ | $-C_2H_5$ | $-N(C_2H_5)_2$ | Öl |
| 21 | $-C_2H_5$ | $-C_2H_5$ | $-SCN$ | 248-250 |
| 22 | $-C_2H_5$ | $-C_2H_5$ | $-J$ | 252 |
| 23 | $-C_2H_5$ | $-C_2H_5$ | $-H$ | 120-123 |
| 24 | $-C_2H_5$ | $-C_2H_5$ | 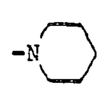 | 132-133 |
| 25 | $-C_2H_5$ | $-C_2H_5$ | $-S-(CH_2)_3-CH_3$ | Öl |
| 26 | $-C_2H_5$ | $-C_2H_5$ | $-OC_2H_5$ | 107-109 |
| 27 | $-C_2H_5$ | $-C_2H_5$ | $-N(CH_3)_2$ | 135-137 |
| 28 | $-C_2H_5$ | $-C_2H_5$ | $-N(n-C_4H_9)_2$ | |

$T = Z = -C_2H_5$

| Beispiel | $A^3$ | Schmelzpunkt [°C] |
|---|---|---|
| 29 | 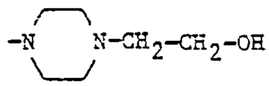 | 154–160 |
| 30 | $-\overset{H}{N}-(CH_2)_2-N(CH_3)_2$ | 110–114 |
| 31 | $-\overset{H}{N}-(CH_2)_2-NH_2$ | 86– 90 |
| 32 | $-CN$ | 103–106 |
| 33 | 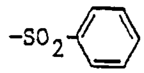 | 237–240 |
| 34 | 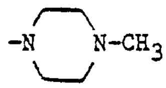 | 93– 95 |
| 35 | 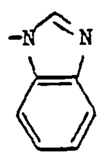 | 103–106 |
| 36 | $-O-CH_2-CH_2-CH_3$ | 107–109 |
| 37 | $-O-(CH_2)_3-CH_3$ | 109–111 |
| 38 | $-O-CH_2-CH_2-O-CH_3$ | 95–97 |
| 40 | $-O-CH_2-C(CH_3)_3$ | 149–151 |
| 41 | 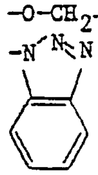 | 90– 93 |
| 42 | 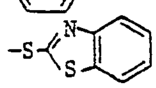 | 133–136 |
| 43 | 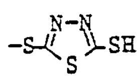 | 89– 92 |
| 44 | 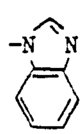 5(6)-$NO_2$ | 218–220 |
| 45 | 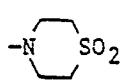 | 234–236 |

II. Anwendung der Triazolylpyrimidine (I)

1. Die in den Anwendungsbeispielen angegebenen xerographischen Meßgrößen A bis G werden wie folgt ermittelt :

Die Schichten werden mit einer Gleichspannungscorona von —7,5 kV in 1 cm Abstand gleichmäßig

auf ein Oberflächenpotential von 600 Volt aufgeladen und dann mit dem weißen Licht einer Xenonlampe mit einer Beleuchtungsstärke von etwa 0,85 mW · $cm^{-2}$ belichtet. Es werden gemessen :

Meßgröße A : Zeit in Millisekunden (ms), innerhalb der das vor Belichtung vorhandene Oberflächenpotential bei aktinischer Belichtung um die Hälfte (300 V) abgefallen ist.

Meßgröße B : Potentialabfall in der gleichen Zeit in Volt (V), der im Dunkeln infolge Dunkelleitfähigkeit der Schichten eintritt.

Meßgröße C : Nach einer Beladungszeit von 20 Sekunden erreichtes Oberflächenpotential in Volt (V) :

Meßgröße D : Potentialabfall in %, bezogen auf Meßgröße C, im Dunkeln innerhalb von 20 Sekunden.

Meßgröße E : Durch die aktinische Belichtung hervorgerufener Potentialabfall in %, bezogen auf das Ausgangspotential unmittelbar vor der Belichtung.

Meßgröße F : Potentialänderung pro Sekunde zu Beginn der Belichtung (V/s).

Meßgröße G : Potentialdifferenz in Volt (V) zwischen belichteten und unbelichteten Flächen der beladenen Schicht.

Anwendungsbeispiele 1 bis 5

Auf eine Polyethylenterephthalatfolie mit einer aufgedampften, leitfähigen Aluminiumschicht in einer Dicke von etwa 300 Å wird eine Schicht aus 60 Teilen eines chlorierten Perylen-3,4 : 9,10-tetracarbonsäurediimidbisbenzimidazols mit einem Chlorgehalt von etwa 38 % und 50 Teilen eines Copolymerisates aus Vinylchlorid, Acrylsäure und einem Maleinsäurediester in einer Dicke von etwa 0,55 μm als Ladungsträger erzeugende Schicht aufgebracht.

Auf diese Ladungsträger erzeugende Schicht wird einer einer Lösung in Essigsäureethylester eine Ladungstransportschicht aus 55 Teilen eines handelsüblichen Bindemittels auf der Basis Polycarbonat mit einem Schmelzbereich von 220 bis 230 °C und jeweils 40 T der in der Tabelle 1 angegebenen Triazolylpyrimidine so aufgebracht, daß nach dem Ablüften und Trocknen (30 min. bei 80 °C) eine Schichtdicke von 12 μm vorhanden ist.

Die erhaltenen Aufzeichnungsmaterialien wurden wie unter II. 1) angegeben geprüft. Die ermittelten Meßgrößen A, B und C sind in der Tabelle 1 zusammengefaßt.

Tabelle 1

Xerographische Meßgrößen der Aufzeichnungsmaterialien

| Anwendungs-beispiel | Triazolyl-pyrimidin aus Beispiel | Meßgröße A [ms] | B [v] | C [v] |
|---|---|---|---|---|
| 1 | 7.3 | 190 | 0,25 | 2100 |
| 2 | 8.3 | 195 | 0,2 | 1600 |
| 3 | 1.3 | 150 | 0,3 | 2000 |
| 4 | 4 | 155 | 0,85 | 1050 |
| 5 | 3 | 170 | 0,2 | 1300 |

Wie die Meßergebnisse zeigen, weisen die elektrophotographischen Aufzeichnungsmaterialien mit den Triazolylpyrimidinen gemäß der vorliegenden Erfindung eine hohe Photoleitfähigkeit und eine niedrige Dunkelleitfähgkeit auf. So zeigt z. B. die Schicht des Anwendungsbeispiels 1 im Dunkeln innerhalb von etwa 0,2 Sekunden einen Potentialabfall von 600 auf 599,8 Volt, während in der gleichen Schicht in der gleichen Zeit bei Belichtung mit einer Beleuchtungsstärke von 0,85 mW · $cm^{-2}$ das Potential von 600 auf 300 Volt abfällt. Die maximale Beladbarkeit ist mit größer 1.500 Volt weit über den in Kopiergeräten erforderlichen Oberflächenpotentialen (ca. 700 Volt), so daß die Schichten für die Kopiertechnik sehr geeignet sind.

Anwendungsbeispiele 6 bis 10

55 Teile eines Copolymerisates aus 70 % Styrol, 6 % Maleinsäureanhydrid und 24 % Acrylsäure mit einem mittleren Molekulargewicht von ca. 2 000, 845 Teile der in der Tabelle 2 angegebenen Triazolylpyrimidine und 0,3 Teile Methylviolett (C.I. 42535) werden in Essigsäureethylester gelöst, und die Lösung wird auf einen elektrisch leitfähigen Träger aus einem elektrolytisch aufgerauhten und danach anodisch oxiderten Aluminiumblech von 0,15 mm Dicke so aufgetragen, daß nach dem Ablüften des Lösungsmittels und Trocknen (30 min. bei 85 °C) eine Schichtdicke von 4 μm resultiert. Die xerographischen Meßgrößen sind in der Tabelle 2 zusammengefaßt.

Vergleichsbeispiele 1 bis 3

Das Aufzeichnungsmaterial wurde wie bei den Anwendungsbeispielen 6 bis 10 hergestellt, jedoch wurde anstelle der dort angegebenen Triazolylpyrimidine folgende Pyrimidinderivate als Ladungsträger transportierende Verbindungen verwendet :

Vergleichsbeispiel Triazolyl [4,5-d] pyrimidin

1 2-(4'-Methoxyphenyl)-5-dimethylamino-7-dodecathio-
2 2-(4'-Methoxyphenyl)-5-dimethylamino-7-morpholino-
3 2-(4'-Methoxyphenyl)-5-dimethylamino-7-cyclohexylamino-.

die an diesen Aufzeichnungsmaterialien gemessenen xerographischen Meßgrößen sind in der Tabelle 2 zusammengefaßt.

Tabelle 2

Xerographische Meßgrößen der Aufzeichnungsmaterialien

| Anwendungsbeispiel | Triazolylpyrimidin aus Beisp. | Meßgröße A [ms] | C [V] | D [%] | E [%] | F [$V \cdot s^{-1}$] | G [V] |
|---|---|---|---|---|---|---|---|
| 6 | 7.3 | 190 | 1700 | 19 | 93 | -6050 | 1260 |
| 7 | 8.3 | 200 | 1550 | 24 | 89 | -5300 | 1070 |
| 8 | 1.3 | 150 | 1600 | 27 | 96 | -6950 | 1100 |
| 9 | 4 | 160 | 1100 | 40 | 97 | -5500 | 640 |
| 10 | 3 | 165 | 1250 | 31 | 94 | -5900 | 820 |
| Vergleichsbeispiel | | | | | | | |
| 1 | | - | 1560 | 38 | 3 | -220 | - |
| 2 | | - | 450 | 36 | 4 | -340 | - |
| 3 | | - | 1280 | 49 | 5 | -200 | - |

Anwendungsbeispiel 11

50 T. eines Copolymerisates aus 60 % Styrol und 40 % einer mit Methanol halbveresterten Maleinsäure mit einem mittleren Molekulargewicht $\bar{M}_w$ von 10 000, 50 T. 2-(4'-Diethylaminophenyl)-5-diethylamino-7-chlor-triazolyl [4,5-d]-pyrimidin (Beispiel 1.3) und 0,2 T. Kristallviolett (C.I. 42555) werden aus einer 5 %igen Lösung in Tetrahydrofuran auf eine elektrolytisch aufgerauhte und anodisierte Aluminiumfolie von 0,15 mm Dicke in einer Trockenschichtdicke von etwa 4 µm aufgebracht.

Diese Druckplatte wird nach einer Aufladung mittels einer Hochspannungscorona in einer Kamera bildmäßig 25 Sekunden belichtet. Danach wird mit einem Pulvertoner entwickelt, der bei 160 °C abriebfest eingebrannt wird. Die unbetonerte Schicht mit mit einem Gemisch aus 05 % Soda, 25 % Isopropanol und 74,5 % Wasser abgewaschen, wodurch die Aluminiumoberfläche freigelegt wird. die Lösungen werden mit einem Wattebausch über die Schicht gestrichen. Man erhält die im Offsetdruck erwünschte Differenzierung in hydrophile und oleophile Bereiche, wobei die Trägeroberfläche die hydrophilen Bereiche liefert.

Anschließend an die Behandlung mit der alkalischen Flüssigkeit wird die Druckplatte mit Wasser nachgespült und durch Überwischen mit verdünnter Phosphorsäurelösung die Hydrophilie der Trägeroberfläche weiter erhöht. Nach Einfärben mit fetter Farbe wird auf bekannte Weise in Offsetdruckmaschinen damit gedruckt.

Anwendungsbeispiel 12

50 Teile des Copolymerisats aus 60 % Styrol, 35 % Methacrylsäure und 5 % Maleinsäureanhydrid (K-Wert : 39,5 ; Erweichungspunkt ca. 225 °C), 50 Teile 2-(4'-Diethylaminophenyl)-5-diethylamino-7-n-butoxy-triazolyl [4,5-d]-pyrimidin (Beisp. 37) und 0,2 Teile C.I. Basic Violet 10, C.I. 45 174 Base (Rhodamin B Base) werden aus einer 5 %igen Lösung in Tetrahydrofuran auf eine elektrolytisch aufgerauhte und

anodisierte Aluminiumfolie von 0,15 mm Dicke in einer Trockenschichtdicke von etwa 4 μm aufgebracht.

Die erhaltene Druckplatte wird dann weiter wie im Anwendungsbeispiel 11, Absätze 2 und 3 angegeben weiterbehandelt. Man erhält eine Druckplatte, die in bekannter Weise auf Offsetdruckmaschinen verwendet werden kann.

Entsprechende Druckplatten erhält man, wenn man anstelle der im Absatz 1 angegebenen Triazolylpyrimidinverbindung eine der folgenden verwendet :

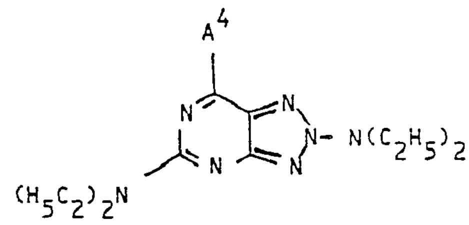

| | |
|---|---|
| $A^4$ = $-OC_2H_5$ | (Beispiel 26) |
| $-OCH_2-CH_2-CH_3$ | (Beispiel 36) |
| $-OCH_2-C(CH_3)_3$ | (Beispiel 40) |
| $-O-CH_2-CH_2-OCH_3$ | (Beispiel 38). |

**Patentansprüche**

1. Neue 2H-v-Triazolyl [4,5-d]-pyrimidine der Formel

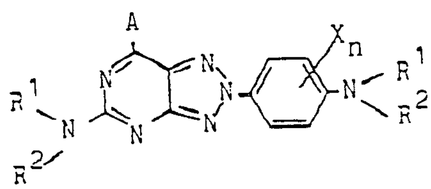 (I)

in der $R^1$ und $R^2$ unabhängig voneinander $C_1$- bis $C_4$-Alkyl, gegebenenfalls durch Chlor, Brom, $C_1$- bis $C_4$-Alkyl oder $C_1$- bis $C_4$-Alkoxy substituiertes Phenyl, Phenalkyl mit insgesamt 7 bis 10 C-Atomen oder

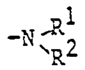

einen gesättigten 5- oder 6-gliedrigen heterocyclischen Ring, und wobei die beiden

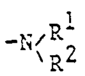

-Gruppen gleich oder verschieden sein können,

X $C_1$- bis $C_4$-Alkyl, Halogen oder Phenyl

n 0, 1 oder 2 und

A 1)

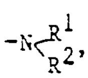,

worin $R^1$ und $R^2$ oder

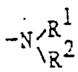

die oben angegebene Bedeutung haben,

2) Wasserstoff, Halogen, Cyan, Thiocyan

3) $C_1$- bis $C_6$-Alkoxy, $C_1$- bis $C_8$-Alkoxy-$C_2$- oder $C_3$-alkoxy, gegebenenfalls durch Cl, Br, $C_1$- bis $C_4$-Alkyl substituiertes Phenoxy, Phenalkoxy mit insgesamt 7 bis 10 C-Atomen, $C_1$- bis $C_{12}$-Alkylthio,

gegebenenfalls durch Chlor, Brom, $C_1$- bis $C_4$-Alkyl und/oder $C_1$- bis $C_4$-Alkoxy substituiertes Phenylthio, $—S—(CH_2)_m—COOR^3$

4) $C_1$- bis $C_6$-Alkylcarbonyl, gegebenenfalls durch $C_1$- bis $C_4$-Alkyl oder $C_1$- bis $C_4$-Alkoxy substituiertes Benzoyl, Carboxyl, Carbo-$C_1$- bis $C_8$-alkoxy,

5) $C_1$- bis $C_4$-Alkylsulfonyl, Phenylsulfonyl

6) $C_5$- bis $C_8$-Cycloalkylamino,

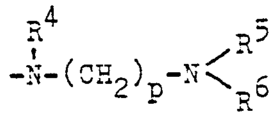 oder 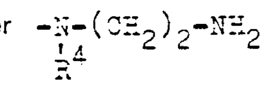

7)

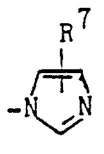 , 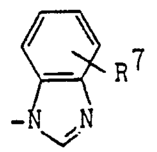 , 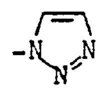 , 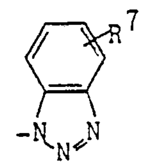

oder

8) 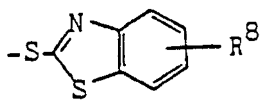 , 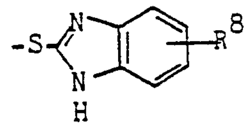 , 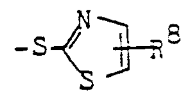

oder 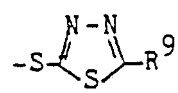

bedeuten und worin

$R^3$ für $C_1$- bis $C_4$-Alkyl,

m für 1, 2, 3 oder 4,

$R^4$ für Wasserstoff oder $C_1$- bis $C_4$-Alkyl,

$R^5$ und $R^6$ unabhängig voneinander für $C_1$- bis $C_4$-Alkyl oder

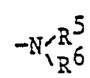

für einen gesättigten 5- oder 6-gliedrigen heterocyclischen Rest, der gegebenenfalls, N, O und/oder S als weiteres Ringglied enthält,

P für 2, 3, 4 oder 5,

$R^7$ und $R^8$ unabhängig voneinander für Wasserstoff, $C_1$- bis $C_4$-Alkyl, $C_1$- bis $C_4$-Alkylcarbonyl, Nitro, Halogen oder Phenyl

und

$R^9$ für Wasserstoff, $C_1$- bis $C_4$-Alkyl, $C_1$- bis $C_4$-Alkylcarbonyl, Phenyl, Thio oder $C_1$- bis $C_4$-Alkylthio stehen.

2. Triazolylpyrimidine gemäß Anspruch 1, dadurch gekennzeichnet, daß $R^1$ und $R^2$ Methyl, Ethyl, Phenyl, Benzyl oder

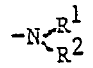

Pyrrolidinyl, Piperidinyl, Morpholinyl oder 4-$C_1$- bis $C_4$-Alkylpiperazinyl bedeuten.

3. Triazolylpyrimidine gemäß Anspruch 1 oder 2, dadurch gekennzeichnet, daß

n für 0 und

A 1.1) für

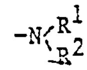

mit $R^1$ und $R^2$ Methyl oder Ethyl oder

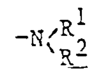

für Morpholinyl, Piperidinyl, Pyrrolidinyl oder N'-$C_1$- bis $C_4$-Alkylpiperazin,

2.1) für Wasserstoff, Chlor,

3.1) für $C_1$- bis $C_6$-Alkoxy, 2-Methoxyethoxy, Phenoxy, Benzyloxy, Butylthio, Phenylthio, $—S—(CH_2)_2—COOR^3$,

4.1) für Acetyl, Propionyl, Benzoyl, Carbomethoxy, Carboethoxy,

5.1) für Phenylsulfonyl

6.1) für

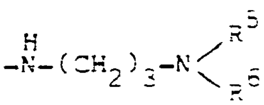 oder 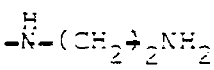,

7.1) für

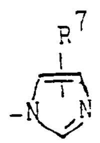, 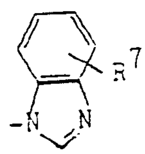,

oder

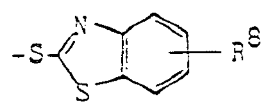,

$R^3$ für $C_1$- bis $C_4$-Alkyl, $R^5$ und $R^6$ für Methyl oder Ethyl und $R^7$ und $R^8$ für Wasserstoff oder $—NO_2$ stehen.

4. Triazolylpyrimidine gemäß Anspruch 2 oder 3, gekennzeichnet durch die Formel

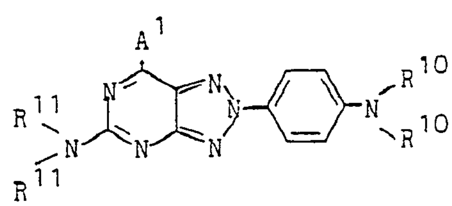

(II)

in der

$A^1$ Chlor, Brom, $—S—(CH_2)_2—COOC_1$- bis $C_4$-Alkyl, N-Morpholino, N-Piperidino, N-Cyclohexylamino, N'-$C_1$- bis $C_4$-Alkylpiperazino, Imidazolyl-(1) oder $C_1$- bis $C_6$-Alkoxy und

$R^{10}$ und $R^{11}$ unabhängig voneinander $C_1$- bis $C_4$-Alkyl oder Phenyl bedeuten.

5. Triazolylpyrimidine gemäß Anspruch 4, dadurch gekennzeichnet, daß

$A^1$ Chlor, $—S—(CH_2)_2—COOCH_3$, N-Morpholino, N-Piperidino, N-Cyclohexylamino, 4-Methylpiperazino, Imidazolyl-(1), Methoxy, Ethoxy, n-Propoxy, n-Butoxy oder 2,2-Dimethylpropoxy und

$R^{10}$ und $R^{11}$ Methyl, Ethyl oder Phenyl bedeuten, wobei die Reste $R^{10}$ und $R^{11}$ gleich oder verschieden sind.

6. Triazolylpyrimidine gemäß Anspruch 5, dadurch gekennzeichnet, daß

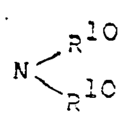 und 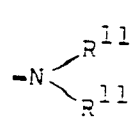

unabhängig voneinander für Dimethylamino, Diethylamino oder Diphenylamino stehen.

7. Triazolylpyrimidine gemäß Anspruch 4, gekennzeichnet durch die Formel

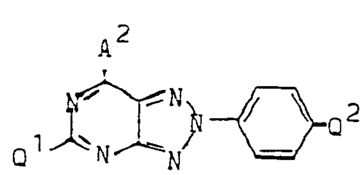

(III)

in der
$A^2$ für Chlor,

$$-S-(CH_2)_2\overset{O}{C}OCH_3,$$

Diethylamino, Methoxy, Ethoxy, n-Propoxy, n-Butoxy oder 2,2-Dimethylpropoxy und $Q^1$ für Diethylamino oder Dimethylamino und $Q^2$ für Diethylamino oder Diphenylamino stehen.

8. Triazolylpyrimidine gemäß Anspruch 7, dadurch gekennzeichnet, daß $A^2$ für Chlor oder Methoxy und $Q^1$ und $Q^2$ für Diethylamino stehen.

9. Verwendung der Triazolylpyrimidine gemäß den Ansprüchen 1 bis 8 als Ladungen transportierende Verbindungen in elektrophotographischen Aufzeichnungsmaterialien.

10. Verwendung der Triazolylpyrimidine gemäß den Ansprüchen 1 bis 8 zur Herstellung von elektrophotographischen Druckplatten, insbesondere von Offsetdruckplatten.

**Claims**

1. A novel 2H-v-triazolo [4,5-d] pyrimidine of the formula

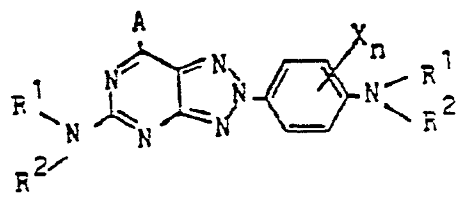

(I)

where

$R^1$ and $R^2$ independently of one another are $C_1$-$C_4$-alkyl, phenyl which is unsubstituted or substituted by chlorine, bromine, $C_1$-$C_4$-alkyl or $C_1$-$C_4$-alkoxy, or phenylalkyl having a total of 7 to 10 carbon atoms,
or

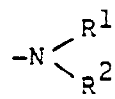

is a saturated 5-membered or 6-membered heterocyclic ring, and the two

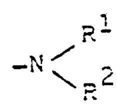

groups can be identical or different,
X is $C_1$-$C_4$-alkyl, halogen or phenyl,
n is 0, 1 or 2, and
A is
1)

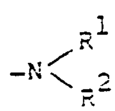

where $R^1$, $R^2$ and

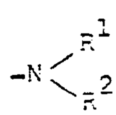

have the above meanings,

2) hydrogen, halogen, cyano or thiocyano,

3) $C_1$-$C_6$-alkoxy, $C_1$-$C_8$-alkoxy-$C_2$- or -$C_3$-alkoxy, phenoxy which is unsubstituted or substituted by Cl, Br or $C_1$-$C_4$-alkyl, phenylalkoxy having a total of 7 to 10 carbon atoms, $C_1$-$C_{12}$-alkylthio, phenylthio which is unsubstituted or substituted by chlorine, bromine, $C_1$-$C_4$-alkyl and/or $C_1$-$C_4$-alkoxy, or $-S-(CH_2)_m-COOR^3$,

4) $C_1$-$C_6$-alkylcarbonyl, benzoyl which is unsubstituted or substituted by $C_1$-$C_4$-alkyl or $C_1$-$C_4$-alkoxy, carboxyl or carbo-$C_1$-$C_8$-alkoxy,

5) $C_1$-$C_4$-alkylsulfonyl or phenylsulfonyl,

6) $C_5$-$C_8$-cycloalkylamino,

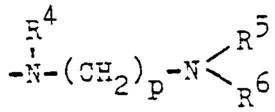 or 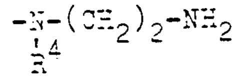

7) 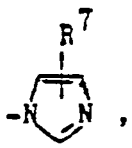 , 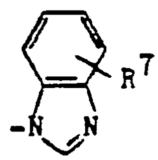 , 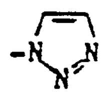 , 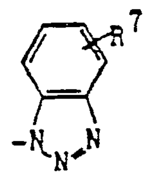

or

8) 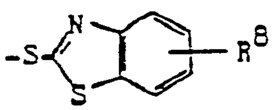 , 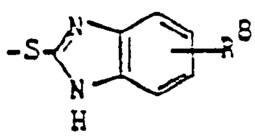 , 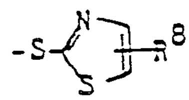

or

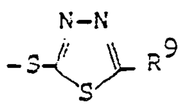

and where

$R^3$ is $C_1$-$C_4$-alkyl,

m is 1, 2, 3 or 4,

$R^4$ is hydrogen or $C_1$-$C_4$-alkyl,

$R^5$ and $R^6$ independently of one another are $C_1$-$C_4$-alkyl, or

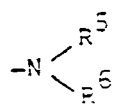

is a saturated 5-membered or 6-membered heterocyclic radical which may or may not contain N, O or S as further ring members,

p is 2, 3, 4 or 5,

$R^7$ and $R^8$ independently of one another are hydrogen, $C_1$-$C_4$-alkyl, $C_1$-$C_4$-alkylcarbonyl, nitro, halogen or phenyl, and

$R^9$ is hydrogen, $C_1$-$C_4$-alkyl, $C_1$-$C_4$-alkylcarbonyl, phenyl, thio or $C_1$-$C_4$-alkylthio.

2. A triazolopyrimidine as claimed in claim 1, wherein $R^1$ and $R^2$ are each methyl, ethyl, phenyl or benzyl,

or

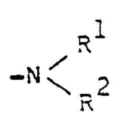

is pyrrolidinyl, piperidino, morpholino or 4-$C_1$-$C_4$-alkylpiperazinyl.

3. A triazolopyrimidine as claimed in claim 1 or 2, wherein

n is 0, and

A is

1.1)

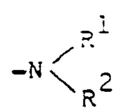

where $R^1$ and $R^2$ are each methyl or ethyl,

or

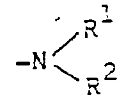

is morpholino, piperidino, pyrrolidinyl or N'-$C_1$-$C_4$-alkylpiperazinyl,

2.1) hydrogen or chlorine,

3.1) $C_1$-$C_6$-alkoxy, 2-methoxyethoxy, phenoxy, benzyloxy, butylthio, phenylthio or $-S-(CH_2)_2-COOR^3$,

4.1) acetyl, propionyl, benzoyl, carbomethoxy or carboethoxy,

5.1) phenylsulfonyl,

6.1)

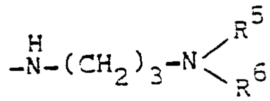 or 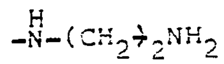

or

7.1)

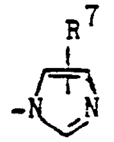 , 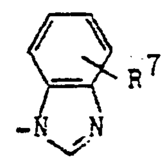

or 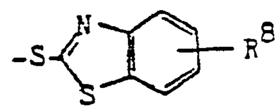 , (II)

where $R^3$ is $C_1$-$C_4$-alkyl, $R^5$ and $R^6$ are each methyl or ethyl, and $R^7$ and $R^8$ are each hydrogen or $-NO_2$.

4. A triazolopyrimidine as claimed in claim 2 or 3, of the formula

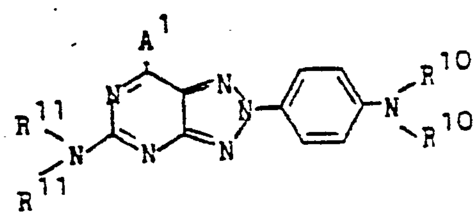

where

$A^1$ is chlorine, bromine, $-S-(CH_2)_2-COOC_1-C_4$-alkyl, N-morpholino, N-piperidino, N-cyclohexylamino, N'-$C_1$-$C_4$-alkylpiperazinyl, imidazol-1-yl or $C_1$-$C_6$-alkoxy, and

$R^{10}$ and $R^{11}$ independently of one another are $C_1$-$C_4$-alkyl or phenyl.

5. A triazolopyrimidine as claimed in claim 4, wherein

$A^1$ is chlorine, $-S-(CH_2)_2-COOCH_3$, N-morpholino, N-piperidino, N-cyclohexylamino, 4-methylpiperazinyl, imidazol-1-yl, methoxy, ethoxy, n-propoxy, n-butoxy or 2,2-dimethylpropoxy, and

$R^{10}$ and $R^{11}$ are identical or different and are each methyl, ethyl or phenyl.

6. A triazolopyrimidine as claimed in claim 5, wherein the radicals

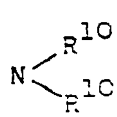 and 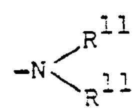

independently of one another, are dimethylamino, diethylamino or diphenylamino.

7. A triazolopyrimidine as claimed in claim 4, of the formula

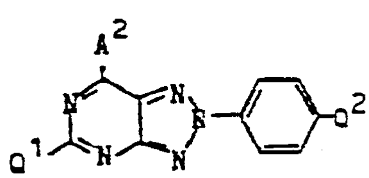

(III)

where $A^2$ is chlorine,

$$-S-(CH_2)_2-\overset{O}{C}OCH_3,$$

diethylamino, methoxy, ethoxy, n-propoxy, n-butoxy or 2,2-dimethylpropoxy, $Q^1$ is diethylamino or dimethylamino, and $Q^2$ is diethylamino or diphenylamino.

8. A triazolopyrimidine as claimed in claim 7, wherein $A^2$ is chlorine or methoxy, and $Q^1$ and $Q^2$ are each diethylamino.

9. The use of a triazolopyrimidine as claimed in claims 1 to 8 as a charge carrier-transporting compound in electrophotographic recording materials.

10. The use of a triazolopyrimidine as claimed in claims 1 to 8 for the production of electrophotographic printing plates, especially offset printing plates.

**Revendications**

1. Nouvelles 2H-v-triazolyl(4,5-d)-pyrimidines de formule

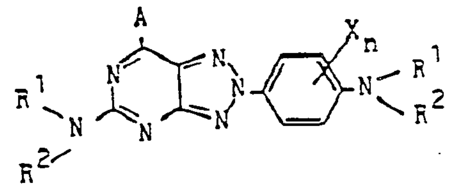

(I)

$R^1$ et $R^2$, considérés indépendamment l'un de l'autre, représentent chacun un radical alkyle en $C_1$ à $C_4$, un radical phényle éventuellement substitué par un atome de chlore, de brome ou par un radical alkyle en $C_1$ à $C_4$ ou alcoxy en $C_1$ à $C_4$, ou un radical phénylalkyle à 7-10 atomes de C au total ou

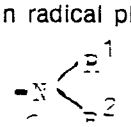

représente un noyau hétérocyclique pentagonal ou hexagonal saturé, les deux groupes

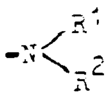

pouvant être semblables ou différents,

X est un radical alkyle en $C_1$ à $C_4$, un atome d'halogène ou un radical phényle,

n est égal à 0, 1 ou 2 et

A représente :

1) un groupement

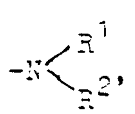

$R^1$ et $R^2$ ou

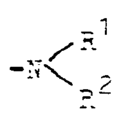

ayant les significations indiquées ci-dessus,

2) un atome d'hydrogène, d'halogène, un groupe cyano ou thiocyano,

3) un radical alcoxy en $C_1$ à $C_6$, $C_1$-$C_8$-alcoxy-$C_2$ ou $C_3$-alcoxy, phénoxy éventuellement substitué par Cl, Br, alkyle en $C_1$ à $C_4$ ; phénalcoxy à 7-10 atomes de C au total ; $C_1$-$C_{12}$-alkylthio, phénylthio éventuellement substitué par un atome de chlore, de brome, par un radical alkyle en $C_1$ à $C_4$ et/ou par un radical alcoxy en $C_1$ à $C_4$, $-S-(CH_2)_m-COOR^3$

4) un groupement $C_1$-$C_6$-alkylcarbonyle, benzoyle éventuellement substitué par un radical alkyle en $C_1$ à $C_4$ ou alcoxy en $C_1$ à $C_4$ ; carboxyle ; carbo-$C_1$-$C_8$-alcoxy,

5) un radical $C_1$-$C_4$-alkylsulfonyle, phénylsulfonyle,

6) $C_5$-$C_8$-cycloalkylamino,

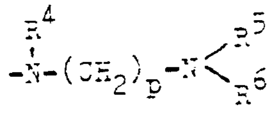 ou 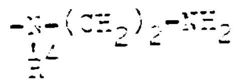

7) 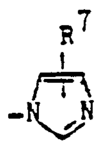 , 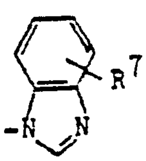 , 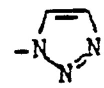 , 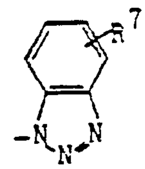

ou

8) 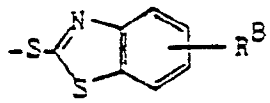 , 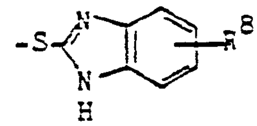 , 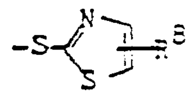

ou 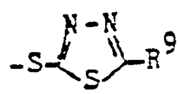

et dans laquelle

$R^3$ est mis pour un radical alkyle en $C_1$ à $C_4$,

m pour le nombre 1, 2, 3 ou 4,

$R^4$ pour un atome d'hydrogène ou un radical alkyle en $C_1$ à $C_4$,

$R^5$ et $R^6$, considérés indépendamment, sont mis chacun pour un radical alkyle en $C_1$ à $C_4$ ou

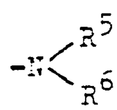

est mis pour un radical hétérocyclique pentagonal ou hexagonal saturé qui contient éventuellement N, O et/ou S en tant que terme cyclique,

p est égal à 2, 3, 4 ou 5,

$R^7$ et $R^8$, considérés indépendamment, sont mis chacun pour un atome d'hydrogène, un groupe alkyle en $C_1$ à $C_4$, un groupe $C_1$-$C_4$-alkylcarbonyle, nitro, un halogène ou un radical phényle et

$R^9$ est mis pour un atome d'hydrogène, un radical alkyle en $C_1$ à $C_4$, $C_1$-$C_4$-alkylcarbonyle, phényle, thio ou $C_1$-$C_4$-alkylthio.

2. Triazolylpyrimidines selon la revendication 1, caractérisées en ce que $R^1$ et $R^2$ représentent chacun un radical méthyle, éthyle, phényle, benzyle ou le groupement

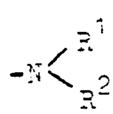

est un groupement pyrrolidinyle, pipéridinyle, morpholinyle ou 4-$C_1$-$C_4$-alkylpipérazinyle.

3. Triazolylpyrimidines selon la revendication 1 ou 2, caractérisées en ce que

n est mis pour 0 et

A est mis pour :

1.1)

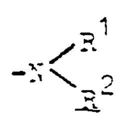

avec $R^1$ et $R^2$ = méthyle ou éthyle ou

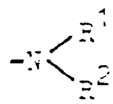

est mis pour un groupement morpholinyle, pipéridinyle, pyrrolidinyle ou N'-$C_1$-$C_4$-alkylpipérazine,

2.1) un atome d'hydrogène ou de chlore,

3.1) un groupe alcoxy en $C_1$ à $C_6$, 2-méthoxyéthoxy, phénoxy, benzyloxy, butylthio, phénylthio, $—S—(CH_2)_2—COOR^3$,

4.1) un groupe acétyle, propionyle, benzoyle, carbométhoxy ou carboéthoxy,

5.1) un groupe phénylsulfonyle,

6.1)

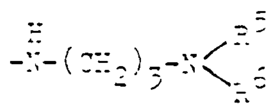 ou 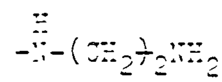

7.1)

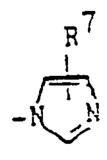 , 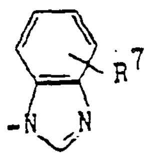 ,

ou

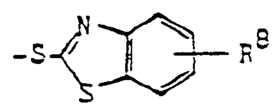 ,

$R^3$ est mis pour un radical alkyle en $C_1$ à $C_4$,

$R^5$ et $R^6$ sont mis chacun pour un radical méthyle ou éthyle et

$R^7$ et $R^8$ sont mis chacun pour un atome d'hydrogène ou $—NO_2$.

4. Triazolylpyrimidines selon la revendication 2 ou 3, caractérisées par la formule

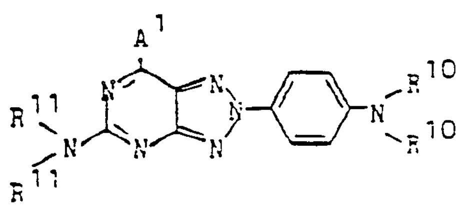

(II)

dans laquelle

$A^1$ représente un atome de chlore, de brome, un groupement $—S—(CH_2)_2—COO$-$C_1$-$C_6$-alkyle, N-morpholino, N-pipéridino, N-cyclohexylamino, N'-$C_1$-$C_4$-alkylpipérazino, imidazolyle-(1) ou alcoxy en $C_1$ à $C_6$ et

$R^{10}$ et $R^{11}$, considérés indépendamment l'un de l'autre, représentent un radical alkyle en $C_1$ à $C_4$ ou phényle.

5. Triazolylpyrimidines selon la revendication 4, caractérisées en ce que

$A^1$ représente un atome de chlore, un groupement $—S—(CH_2)_2—COOCH_3$, N-morpholino, N-pipéridino, N-cyclohexylamino, 4-méthylpipérazino, imidazolyle-(1), méthoxy, éthoxy, n-propoxy, n-butoxy ou 2,2-diméthylpropoxy et

$R^{10}$ et $R^{11}$ sont mis chacun pour un radical méthyle, éthyle ou phényle, les radicaux $R^{10}$ et $R^{11}$ étant semblables ou différents.

6. Triazolylpyrimidines selon la revendication 5, caractérisées en ce que

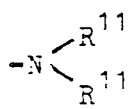 et 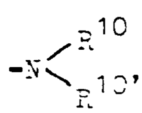,

indépendamment l'un de l'autre, représentent chacun un groupement diméthylamino, diéthylamino ou diphénylamino.

7. Triazolylpyrimidines selon la revendication 4, caractérisées par la formule

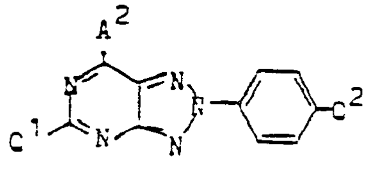

(III)

dans laquelle $A^2$ est mis pour un atome de chlore, un groupement

$$-S-(CH_2)_2\overset{O}{\overset{\|}{C}}OCH_3,$$

diéthylamino, méthoxy, éthoxy, n-propoxy, n-butoxy ou 2,2-diméthylpropoxy et $Q^1$ est mis pour un groupement diéthylamino ou diméthylamino et $Q^2$ pour un groupement diéthylamino ou diphénylamino.

8. Triazolylpyrimidines selon la revendication 7, caractérisées en ce que $A^2$ est mis pour un atome de chlore ou un groupement méthoxy et $Q^1$ et $Q^2$ pour un groupement diéthylamino.

9. Utilisation des triazolylpyrimidines selon l'une quelconque des revendications 1 à 8 comme composés transporteurs de charges dans des matériels d'enregistrement électrophotographique.

10. Utilisation des triazolylpyrimidines selon l'une quelconque des revendications 1 à 8 pour la fabrication de formes d'impression électrophotographique, en particulier de formes d'impression offset.